# EUROPÄISCHE PATENTSCHRIFT

(11) **EP 2 065 387 B1**
(45) Veröffentlichungstag und Bekanntmachung des Hinweises auf die Patenterteilung: **19.01.2011**
(21) Anmeldenummer: 08019467.3
(22) Anmeldetag: 07.11.2008
(51) Int. Cl.: C07D 491/22, B01J 19/12

(54) **Quarzglas-Mikrophotoreaktor und Synthese von 10-Hydroxycamptothecin und 7-Alkyl-10-hydroxycamptothecin**
Quartz glass micro-photoreactors and synthesis of 10-hydroxycamptothecin and 7-alkyl-10-hydroxycamptothecin
Microphotoréacteur en verre à quartz et synthèse de 10-hydroxycamptothécine et 7-alkyl-10-hydroxycamptothécine

(30) Priorität: 29.11.2007 DE 102007057869
(43) Veröffentlichungstag der Anmeldung: 03.06.2009
(73) Patentinhaber: W.C. Heraeus GmbH, 63450 Hanau (DE)
(72) Erfinder: Werner, Silvia, 63796 Kahl (DE); Seliger, Raimund, 63579 Freigericht (DE); Rauter, Holger, Dr., 36103 Flieden (DE); Wissmann, Friedrich, Dr., 63755 Alzenau (DE)
(74) Vertreter: Kühn, Hans-Christian

(56) Entgegenhaltungen:
- EP-A1- 0 074 256
- WO-A1-2006/087353
- DE-A1- 10 209 898
- JP-A- 2006 290 765
- US-A- 5 779 912
- MATTHIAS BOHNET ET AL: "ULLMANN'S ENCYCLOPEDIA OF INDUSTRIAL CHEMISTRY SIXTH COM. REV. ED. VOL. 26, chapter 3.1 "Photoreactor design"" ULLMANN'S ENCYCLOPEDIA OF INDUSTRIAL CHEMISTRY, Bd. 1, 1. Januar 2003 (2003-01-01), Seiten 248-251, XP002524430
- ANONYMOUS: "NORMAG 2005: Fallfilm - Photoreaktor mit Flüssigkeitszwangsumwälzung" NORMAG - LABORGLASGERÄTEBAU, 2005, Seiten 12.1-12.10, XP002524431

## Beschreibung

Die Erfindung betrifft Verfahren zur Herstellung von 10-Hydroxycamptothecin und von 7-Alkyl-10-hydroxycamptothecin und Vorrichtungen zur Ausführung der Verfahren.

### Hintergrund

EP 0 074 256 A1 ("Camptothecin derivatives, process for preparing same, formulations containing such derivatives and their use") und US 4,545,880 ("Photochemical process for preparing Camptothecin derivatives", Beispiel 7) beschreiben die Herstellung von 10-Hydroxycamptothecin und 7-Alkyl-10-hydroxycamptothecin durch Bestrahlung von Camptothecin-*N*-oxid bzw. 7-Alkyl-camptothecin-*N*-oxid in einem Lösemittel, bevorzugt Dioxan, Acetonitril, Chloroform, Dichlormethan, Glyme, Diglyme und Mischungen daraus, unter Zusatz von Wasser in Gegenwart einer Säure, bevorzugt Mineralsäuren, organischer Sulfonsäuren, Lewis-Säuren und organischer Carbonsäuren. Die Bestrahlung der Reaktionsmischung mit UV-Licht erfolgt entsprechend dem damals bekannten Stand der Technik unter Einsatz einer Quecksilberlampe.

In EP 0 074 256 A1 wird beschrieben, dass die Reaktion der *N*-Oxide zu zwei verschiedenen Reaktionsprodukten führt, nämlich das an 1-Position deoxygenierte Startmaterial (Camptothecin oder 7-Alkylcamptothecin) und die gewünschten 10-Hydroxy-Derivate. O-Hydroxycamptothecin wurde aus Camptothecin-*N*-oxid durch Photolyse in Dioxan/MeCN/H₂O (5:4:1) in Gegenwart von H₂SO₄ mit 88.2% Ausbeute erhalten.

Lei et al., Chemical Research in Chinese Universities, 17 (1), 69-72 (2001), beschreiben ausgehend von Camptothecin (CAS 7689-03-4) ein dreistufiges Verfahren, das zu 7-Ethyl-10-hydroxycamptothecin (SN-38) führt, wobei in der letzten photochemischen Stufe mit einer Hg-Hochdrucklampe bestrahlt wird.

JP2006290765A ("Method for the production of 7-alkyl-10-hydroxycamptothecins") betrifft die Synthese von 7-Alkyl-10-hydroxycamptothecinen aus 7-Alkylcamptothecin-*N*-oxiden, wobei in Gegenwart von Säuren mit UV-Licht bestrahlt wird. Die Reaktionszeit wird mit 100 min und die Ausbeute mit 79.2 % angegeben. Auf die Bildung der Nebenprodukte (Camptothecin oder 7-Alkylcamptothecin) und damit eine verringerte Ausbeute wird eingegangen und als Lösung für deren Reduzierung wird der Einsatz von UV-Strahlen, deren Wellenlängenbereich so ausgelegt ist, dass keine Wellenlängen kürzer als 370 nm emittiert werden, vorgeschlagen. In einer bevorzugten Ausführung wird mit Licht im Bereich von 370 bis 480 nm (Niederdruck Quecksilberlampe 370-480 nm, Metallhalogenid Lampe 400-430 nm, Light Emitting Diode 405 nm, Laser Diode 405 nm) bestrahlt.

Durch Anwendung des Bestrahlungsspektrums 370 bis 480 nm konnte der Gehalt an 7-Ethylcamptothecin gemäß JP 2006290765 von etwa 12 % auf bis zu 3.8 % erniedrigt werden. Eine Ausbeutesteigerung von etwa 50 % auf 80 % konnte durch Anwendung eines Durchflussrohrreaktors anstelle eines Batch-Reaktors erzielt werden.

Es stellt sich die Aufgabe, ein Verfahren bereitzustellen, das für die industrielle Herstellung von 10-Hydroxycamptothecin und 7-Alkyl-10-hydroxycamptothecinen geeignet ist. Darüber hinaus ist eine Aufgabe der vorliegenden Erfindung, für die photochemische Herstellung von Camptothecin-Derivaten einen geeigneten Reaktor zu entwickeln, der die Herstellung solcher Produkte und ähnlicher Verbindungen unter reproduzierbaren, ökonomischen und für den Anwender einfachen Bedingungen im industriellen Maßstab erlaubt.

### Detaillierte Beschreibung: Verfahren

Die Erfindung betrifft Verfahren zur Herstellung von 10-Hydroxycamptothecin sowie von 7-Alkyl-10-hydroxycamptothecin, mit den Schritten
**A.** Bereitstellung eines kontinuierlichen - bevorzugt quaderförmigen und/oder beidseitig bestrahlten - Fluidstroms mit Dicke **d** einer Lösung von Camptothecin-*N*-oxid bzw. 7-Alkyl-camptothecin-*N*-oxid;
**B.** Vorbeiführen des Fluidstroms an Lichtquellen, die parallel zur Dicke **d** strahlen, wobei **d** bei 20 µm bis 1000 µm liegt.

Bevorzugt liegt **d** bei 40 µm bis 100 µm. Der Fluidstrom ist mit Vorteil quaderförmig (Länge **I**, Breite **b,** Dicke **d**). Bevorzugt wird von beiden Seiten parallel zur Dicke **d** bestrahlt. **I**n einer ebenfalls bevorzugten Ausführungsform wird mit Licht im Bereich von 350 bis 400 nm bestrahlt.

Geeignete Lichtquellen dafür sind z. B. Niederdruck-Quecksilberlampen mit geeigneten Spektralfilter (350-400 nm), LEDs (lichtemittierende Dioden (375 nm bzw. 385 nm).

Gemäß der vorliegenden Erfindung kann mit einer ebenen, von beiden Seiten bestrahlten Durchflussgeometrie und Nutzung von LED-Strahlungsquellen mit einer Wellenlänge von 385 nm oder Hg-Hochdruck-Strahlern mit Spektralfilter (350nm bis 400nm) der Gehalt an 7-Alkylcamptothecin-Nebenprodukten stark reduziert werden.

Weiterhin kann gemäß der vorliegenden Erfindung durch Einsatz von Dimethylformamid (DMF) oder DMF/Dioxan als Lösemittel die Konzentration der zu bestrahlenden Lösungen von 7-Alkylcamptothecin-*N*-Oxid von bisher beschriebenen 0.1 % auf bis zu 3 % und darüber hinaus gesteigert werden.

Weiterhin konnte gefunden werden, dass bei Durchführung der Bestrahlung mit einem temperierten Fluidstrom von Camptothecin-*N*-oxid bzw. 7-Alkylcamptothecin-*N*-oxid das Nebenproduktspektrum deutlich verbessert werden kann. Im bevorzugten Fall liegt die Temperatur des Fluidstroms bei 40 - 80 °C.

Weiterhin wurde gefunden, dass bei zweiseitiger Bestrahlung Lösungen von Camptothecin-*N-*Oxid und 7-Alkylcamptothecin-*N*-Oxid mit einer höheren Konzentration, bei einer größeren bestrahlten Schichtdicke d immer noch so vollständig durchstrahlt werden, dass eine Umsatzrate von größer 99 % bei einem hohen Fluss erzielt werden kann.

Die folgenden Beispiele erläutern das Verfahren näher. Teile und Prozentangaben beziehen sich wie auch in der übrigen Beschreibung auf das Gewicht, sofern nicht anders angegeben.

### Vergleichsbeispiel 1:

Photochemische Bestrahlung einer 0.1 %igen Lösung von 7-Ethyl-camptothecin-N-oxid zur Herstellung von 7-Ethyl-10-hydroxycamptothecin in einem Photoreaktor (Falling film-Photoreaktor nach Dr. de Meijere; Vorratsgefäß 1000 ml) mit Hg-Hochdruckstrahler ausgelegt als Tauchlampe (Heraeus Noblelight, Typ TQ 718 Z 2; ca. 10 % der Gesamtleistung emittieren im Bereich von 350 bis 400 nm).

7-Ethylcamptothecin-*N*-oxid (0.75 g,1.91 mmol) wird in einem Gemischaus 1,4-Dioxan (730 ml) und 1N Schwefelsäure (1,75 ml) gelöst und in einem Photoreaktor mit Hg-Tauchlampe für 30 min bestrahlt. Die bestrahlte Lösung wird anschließend im Vakuum aufkonzentriert und unter Rühren mit t-Butylmethy-lethylether (10 ml) suspendiert, filtriert und mit Diisopropylether (10 ml) nachgewaschen. Das Produkt wird bei 50 °C im Vakuum getrocknet. Das Rohprodukt, verunreinigt mit 11 % 7-Ethylcamptothecin wird mittels Säulenchromatographie (Kieselgel; Chloroform: Methanol 90:10) gereinigt. Saubere Fraktionen werden aufkonzentriert, filtriert und mit t-Butylmethylether (10 ml) nachgewaschen. Das Produkt wird im Vakuum bei 50 °C getrocknet. Ausbeute: 0.37 g (49 %).

### Beispiel 1:

Photochemische Bestrahlung einer 0.3 %igen Lösung von 7-Ethyl-camptothecin-*N*-oxid zur Herstellung von 7-Ethyl-10-hydroxycamptothecin in einem Mikrophotoreaktor Typ A mit Hochleistungs-LED Array (Fa. Epitex; Typ L 385-30 mit einer typischen Emmissionswellenlänge von 285 nm und 150 mW Strahlungsleistung).

7-Ethylcamptothecin-*N*-oxid (150 g, 0.382 mol) wird in einem Gemisch aus 1,4-Dioxan (46 l), N,N-Dimethylformamid (2.7 l) Wasser (38 g) und konzentrierter Schwefelsäure (38 g) gelöst und über zwei seriell angeordnete Mikrophotoreaktoren vom Typ A bei einer Flussrate von 600 ml/h und einer Fluidtemperatur von 70 -75°C gefördert. Die bestrahlte Lösung wird anschließend im Vakuum auf 200 ml eingeengt. Das erhaltene Gemisch wird dann unter Rühren bei 15 bis 30°C in Wasser (6.3 l) gegeben und nachgerührt. Das Produkt wird filtriert, dreimal mit Wasser (1.5 l) gewaschen und bei 50°C im Stickstoffstrom getrocknet. Gehalt an 7-Ethyl-camptothecin 2.8 %. Ausbeute: 127 g (85 %).

### Beispiel 2:

Photochemische Bestrahlung einer 0.6 % igen Lösung von 7-Ethylcamptothecin-N-oxid zur Herstellung von 7-Ethyl-10-hydroxycamptothecin in einem Quarzglasmikrophotoreaktor Typ B mit Hg-Hochdruck-Strahlern (Heraeus Noblelight, medizinische UV-Einheit) und Interferenz-Filtern mit spezifiziertem Bandpass sowie beidseitiger Beschichtung; Emissionswellenlängenbereich im UV von 350nm bis 400nm.

7-Ethylcamptothecin-*N*-oxid (150 g, 0.382 mol) wird in einem Gemisch aus 1,4-Dioxan (23.1 l), *N,N*-Dimethylformamid (1.4 l) Wasser (38 g) und konzentrierter Schwefelsäure (38 g) gelöst und über einen Quarzglas-Mikrophotoreaktor Typ B bei einer Flussrate von 900 ml/h bei einer Fluidtemperatur von 70 -75°C gefördert. Die bestrahlte Lösung wird anschließend im Vakuum auf 200 ml aufkonzentriert. Das erhaltene Gemisch wird dann unter Rühren in Wasser (6.3 l) gegeben. Das Produkt wird filtriert, dreimal mit Wasser (1.3 l) gewaschen und bei 50°C im Stickstoffstrom getrocknet. Ausbeute: 143 g (95 %).

### Beispiel 3:

Photochemische Bestrahlung einer 0.25 % igen Lösung von Camptothecin-*N*-oxid zur Herstellung von 10-Hydroxycamptothecin in einem Mikrophotoreaktor Typ A mit Hochleistungs-LED Arrays (Fa.Epitex, Typ L 385-30 , typische Emissionswellenlänge von 385nm und einer Strahlungsleistung von 150 mW).

Camptothecin-*N*-oxid (2.5 g, 0.004 mol) wird in einem Gemisch aus 1,4-Dioxan (833 ml), *N,N-*Dimethyl-formamid (167 ml), Wasser (2.5 ml) und konzentrierter Schwefelsäure (1.5 ml) bei 550 °C gelöst und über zwei seriell angeordnete Mikrophotoreaktoren vom Typ A bei einer Flussrate von 480 ml/h bei einer Fluidtemperatur von 70 -75°C gefördert. Die bestrahlte Lösung wird anschließend im Vakuum aufkonzentriert und unter Rühren in Wasser (200 ml) gegeben. Das Produkt wird filtriert, viermal mit Wasser (je 25 ml) gewaschen und bei 40°C im Vakuum getrocknet. Gehalt an Camptothecin 4.3 %¹. Ausbeute: 1.75 g (70 %).
¹ Das Startmaterial enthält etwas 1 % Camptothecin.

### Beispiel 4:

Photochemische Bestrahlung einer 0.45 % igen Lösung von Camptothecin-*N*-oxid zur Herstellung von 10-Hydroxycamptothecin in einem Quarzglasmikrophotoreaktor Typ B Hg-Hochdruck-Strahlern (Heraeus Noblelight) und Interferenz-Filtern mit spezifiziertem Bandpass sowie beidseitiger Beschichtung; Emissinonswellenlängenbereich im UV von 350nm bis 400nm.

Camptothecin-*N*-oxid (4.5 g, 0.012 mol) wird in einem Gemisch aus 1,4-Dioxan (833 ml), *N,N-*Dimethyl-formamid (167ml), Wasser (4.5 ml) und konzentrierter Schwefelsäure (2.7 ml) bei 50 °C gelöst und über einen Quarzglas-Mikrophotoreaktor Typ B bei einer Flussrate von 720 ml/h und einer Fluidtemperatur von 70 - 75°C gefördert. Die bestrahlte Lösung wird anschließend im Vakuum aufkonzentriert und unter Rühren in Wasser (500 ml) gegeben. Das Produkt wird filtriert, mit Wasser gewaschen und bei 40°C im Vakuum getrocknet. Ausbeute: 4.2 g (93 %).

### Detaillierte Beschreibung: Vorrichtungen

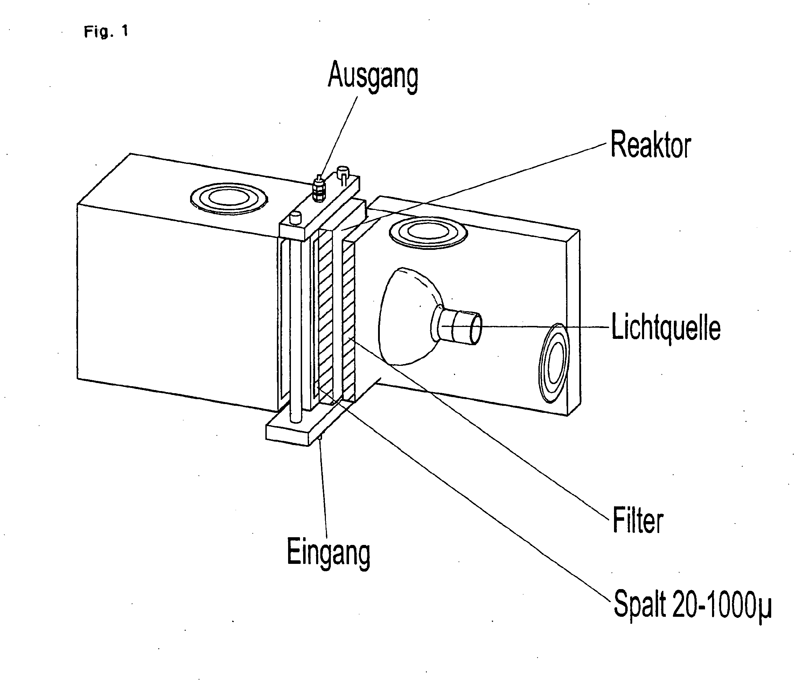

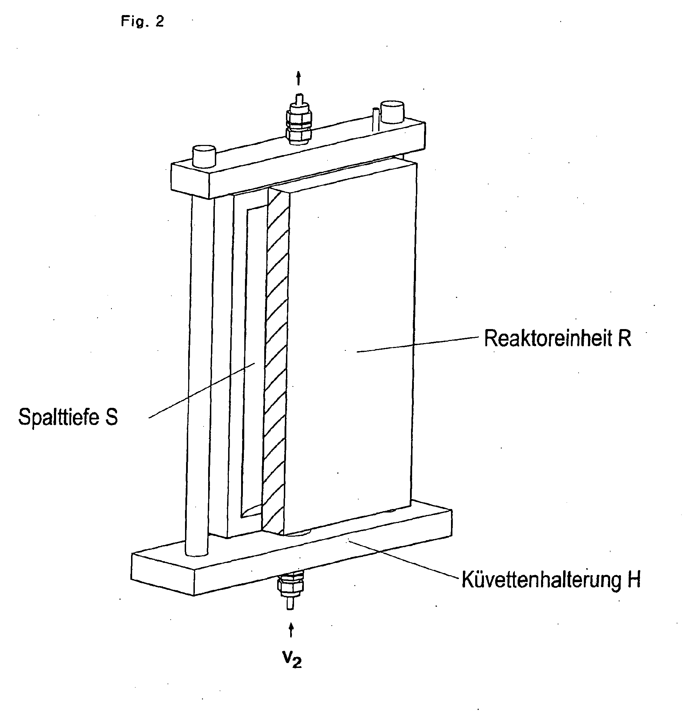

Überraschenderweise wurde gefunden, dass das Strömungsverhalten in einer in ihren Dimensionen stark vergrößerten quaderförmigen Quarzglasdurchflusszelle (siehe Abb. 1) eine ausreichend homogene Durchströmung bei genau einstellbarem Verweilzeitverhalten der Lösung erlaubt. Daher eignet sich eine so konzipierte Durchflusszelle als einfach herzustellender Photoreaktor.

Eine erfindungsgemäß geeignete Vorrichtung ist eine in ihren Eigenschaften verbesserte Ausführung eines Mikrophotoreaktors in Form einer Glasdurchflussküvette (Quarzglasmikrophotoreaktor Typ B) mit minimierter Spaltbreite, die so gefertigt ist, dass sie aus zwei fest miteinander verbundenen, transparenten, ebenen Scheiben besteht. Diese in definiertem Abstand voneinander verbundenen Scheiben bilden einen Spalt, durch den die zu bestrahlende Lösung über einen Zulauf und Ablauf transportiert werden kann. Von außen lässt sich die Glasküvette mit Lichtquellen, die Licht geeigneter Wellenlänge emittieren, bestrahlen. Die Lichtquelle kann so in einer Bestrahlungseinheit integriert sein, dass Filterscheiben zum Einsatz kommen, die unerwünschte Wellenlängenbereiche herausfiltern.

Im Unterschied zu bekannten Mikroreaktoren werden die transparenten Platten des erfindungsgemäßen Quarzglasmikrophotoreaktors nicht kraft- oder formschlüssig, sondern stoffschlüssig miteinander verbunden. Dies hat den wesentlichen Vorteil, dass es die Einstellung einer definierten Spaltbreite reproduzierbarer und präziser erlaubt.

Werden zwei Platten kraft- oder formschlüssig miteinander verbunden, ist die Nutzung zweier transparenter, aus Glas bestehender Teile nicht bevorzugt oder ungünstig. Bei der Einstellung einer eng definierten Spaltbreite ist in jedem Fall eine Kraft senkrecht zu den Platten aufzubringen, die zur Erreichung des Spaltmasses ausreichend ist. Durch diese Krafteinwirkung kann es zu Schaden an den Glasteilen kommen. Zwischen den Glasteilen sind in diesem Fall dann elastische Materialien zu verwenden, die wiederum eine genaue Einstellung der Spaltbreite deutlich erschweren.

Eine stoffschlüssige Verbindung erlaubt dagegen eine druckfreie Herstellung eines Spaltes durch entsprechende Verbindung zweier ebener Platten und dazwischen liegender Stege.

In DE 10341500 A1 wird die Eindringtiefe und damit die Spaltbreite eines Photoreaktors über das Lambert-Beersche Gesetz hergeleitet. Ein von zwei Seiten bestrahlter Quarzglasmikrophotoreaktor ergibt aus einsichtlichen Gründen eine doppelt so große Eindringtiefe des Lichts in das zu bestrahlende Medium, daher kann mit größeren und einfacher herzustellenden Spaltbreiten gearbeitet werden. Andererseits kann bei gleicher Spaltbreite auch mit Lösungen einer höheren Eduktkonzentration gearbeitet werden, was deutliche Einsparungen an Kosten für Lösemittel bewirkt.

Typische Spaltbreiten des hier beschriebenen Quarzglasmikrophotoreaktors, hergestellt aus einer Quarzglasdurchflussküvette, betragen 20 µm bis 1000 µm, bevorzugte Spaltbreiten liegen bei 40 µm bis 100 µm.

Ein wichtiger Bestandteil eines Mikrophotoreaktors ist seine Lichtquelle und deren konstruktive Anordnung im System. Um eine gegenseitige negative Beeinflussung von Reaktionsmedium und Lichtversorgung möglichst auszuschließen, ist die Anordnung der Lichtquelle in einer räumlich getrennten Bestrahlungseinheit von Vorteil.

Bewährt hat sich ein Quarzglasmikrophotoreaktor Typ B mit Hg-Hochdruck-Strahlern (Heraeus Noblelight) und Spektralfiltern mit spezifiziertem Bandpass sowie beidseitiger Beschichtung; Emissionswellenlängenbereich im UV von 350 nm bis 400 nm; Leistung 500 W: Der Hg-Strahler kann ebenso mit Metallhalogenidionen belegt sein.

Mit einem einzigen solchen Reaktor bezogen können etwa 130 g pro Tag an 7-Ethylcamptothecin-*N*-oxid umgesetzt werden. Durch Einsatz entsprechend parallel arbeitender Reaktorsysteme (Numbering up) lassen sich so Mengen im kg-Bereich pro Tag industriell produzieren. Es ist auch möglich, jeweils zwei Reaktoren seriell hintereinander zu schalten.

Mikroreaktoren, wie auch der hier beschriebene Quarzglasmikrophotoreaktor, können verstopfen, wenn Edukte, Zwischen- oder Endprodukte aus dem Reaktionsmedium auskristallisieren oder sich amorph abscheiden. Dies führt zu Undichtigkeiten oder sogar zum Bersten der Reaktoren. Hierdurch tritt Reaktionsmedium oder darin gelöste Stoffe in Kontakt mit den Lichtquellen, wobei direkt auf dem Reaktor angebrachte Lichtquellen Schaden nehmen können.

Erfindungsgemäß sind die Strahlungsquellen daher bevorzugt in einer separaten, abgeschlossenen Bestrahlungseinheit untergebracht, die in definiertem Abstand und Winkel von der zu bestrahlenden Reaktorfläche installiert werden kann.

Eine bevorzugte Ausführungsform einer solchen räumlich getrennten Bestrahlungseinheit besteht aus einer Einhausung aus geeignetem Konstruktionsmaterial, z.B. einem metallischem Werkstoff, in geeigneter Form, z.B. quaderförmig, zylindrisch, kegelförmig oder ähnlich, die die Strahlungsquelle nach allen Seiten außer zum Reaktor hin umschließt. Zur Reaktorseite hin ist diese Bestrahlungseinheit durch eine transparente Platte abgeschlossen.

Diese transparente Platte kann in einer weiteren bevorzugten Ausführungsform als Spektralfilter ausgeführt sein, so dass nur Licht der gewünschten Wellenlänge im UV-, sichtbaren und / oder IR-Bereich auf den Reaktor emittiert wird.

In einer weiteren Ausführungsform kann der Spektralfilter zur Erreichung der gewünschten Wellenlänge im UV-, sichtbaren und/oder IR-Bereich direkt auf die Glasscheiben des Mikrophotoreaktors aufgebracht werden.

Licht im IR-Bereich kann insbesondere dann nützlich sein, wenn mit der Lichtquelle auch gleichzeitig eine Beheizung des Photoreaktors und des durchströmenden Mediums erzielt werden soll.

Eine andere Möglichkeit, das Reaktionsmedium zu erwärmen, besteht in dem Aufbringen einer dünnen Schicht von Indiumzinnoxid oder anderen Beschichtungsmaterialien mit transparentleitfähigen Eigenschaften auf die Außenfläche des Quarzglasmikrophotoreaktors. In jeder Bestrahlungseinheit ist mindestens eine Strahlenquelle installiert. Die Anzahl der Strahlungsquellen richtet sich nach der erforderlichen Bestrahlungsleistung für die durchzuführende Photoreaktion und danach, ob verschiedene Quellen mit unterschiedlichen Wellenlängenemissionen von Vorteil sind.

In einer bevorzugten Ausführungsform werden Gasentladungslampen oder Halbleiterlichtquellen eingesetzt.

### Beschreibungen der getesteten Photoreaktoren

### Photoreaktor mit Hg-Tauchlampe

Der Photoreaktor (Falling-film-Photoreaktor nach Dr. de Meijere) besteht aus einem Bestrahlungsgefäß (200 ml Volumen), mit einem silberverspiegelten Hochvakuummantel mit Sichtstreifen und Temperiermantel. Im Bestrahlungsgefäß befinden sich das Kühlrohr aus Quarglas sowie das Tauchrohr aus Bohrsilikatglas. Die Umwälzung der Flüssigkeit geschieht mit einer Zwangsumwälzpumpe System Normag, die über ein Regelgerät gesteuert wird. Das Vorratsgefäß hat ein Volumen von 1000 ml. Als Strahlungsquelle dient eine Hg-Hochdrucklampe TQ 718 Z2 (Heraeus Noblelight), ausgelegt als Tauchlampe, belegt mit Metallhalogenid-Zusätzen. Dieser Hg-Strahler emittiert das charakteristische Hg-Linienspektrum, welches vom kurzwelligen UV-Bereich von etwa der Wellenlänge 240 nm bis weit in das sichtbare Gebiet reicht. Innerhalb dieses Bereiches liegen einige intensive und mehrere schwächere Linien. Die stärkste Linie innerhalb des UV-Bereiches liegt bei der Wellenlänge 366 nm. Auch die anschließenden Linien im sichtbaren Bereich zwischen 400 und 600 nm sind vielfach bei photochemischen Reaktionen wirksam.

### Mikrophotoreaktor Typ A

Der Photoreaktor besteht aus den Baugruppen Reaktoreinheit und Strahlereinheit. Die Reaktoreinheit besteht im Wesentlichen aus einem massiven Edelstahlblock (235 mm x 210 mm x 22 mm) und einer Glasplatte (205 mm x 150 mm x 10 mm), gehaltert in einer Metallfassung. Der Edelstahlblock dient der Prozessfluidführung. Außerhalb der Fluidzone befindet sich zusätzlich eine 20 µ Dartek®-Unterlage. Auf diese Dartek®-Unterlage wird die Glasplatte aufgepresst. Die Temperierung der Prozessfluidlösung erfolgt über einen separaten Strömungskreislauf. Die Strahlungsquelle besteht aus einem Arrayblock von 50 Hochleistungs-UV-LED-Arrays mit einer typischen Emissionswellenlänge von 385 nm und 150 MW Strahlungsleistung pro Hochleistungs-UV-LED.Array. Die Strahlereinheit wird mit Stickstoff gespült, um einerseits Kondensation von an den teilweise gekühlten elektrischen Komponenten zu vermeiden, anderseits das Eindringen von ggf. entzündlichen Gasgemsichen in die Baugruppe von außen zu verhindern. Die Strahlungsquelle wird korrekt auf dem Reaktorteil positioniert.

### Quarzglasmikrophotoreaktor Typ B

Der Quarzglasmikrophototreaktor besteht aus der Reaktoreinheit R und zwei separat abgeschlossene Bestrahlungseinheiten (Strahlungsquelle).

Die Reaktoreinheit R besteht im Wesentlichen aus zwei Quarzglasplatten P1 und P2. Die Spalttiefe S ist definiert durch einen Abstandshalter, welcher ebenso aus Quarzglas gefertigt ist. Die Quarzplatten P1, P2 und Abstandshalter sind stoffbündig miteinander verbunden.

Der Fluidstrom fließt mit einer Durchflussgeschwindigkeit v₂ durch eine Bohrung und kann, bevor er in die bestrahlte Zone kommt, einen Eintrittskanal durchlaufen.

Jede Bestrahlungseinheit (Strahlungsquelle) besteht aus einem Hg-Hochdruck-Strahler (Heraeus Noblelight) und einem Spektralfilter mit spezifiziertem Bandpass sowie beidseitiger Beschichtung, so dass der Emissinonswellenlängenbereich im UV von 350 nm bis 400 nm erreicht wird. Die Leistung des Hg-Hochdruck-Strahlers beträgt 500 W. Der Hg-Strahler kann ebenso mit Metallhalogenidionen belegt sein.

Die IR-Strahlung des Hg-Hochdruck-Strahlers wird zur Heizung des Fluidstroms verwendet.
Fig. 1 zeigt einen erfindungsgemäßen zweiseitig bestrahlten Quarzglasmikrophotoreaktor Typ B mit Reaktoreneinheit und zwei Strahlungsquellen. Die Strahlungsquellen sind bei der in Fig. 1 gezeigten Vorrichtung in je einer separaten, abgeschlossenen Bestrahlungseinheit untergebracht.
Fig**.** 2 zeigt die Reaktoreneinheit R mit Ein- und Ausgang (Fig. **4** **6,7)** des Fluidstroms mit Durchflussgeschwindigkeit v₂.
Fig. 3 zeigt die schematische Anordnung eines zweiseitig bestrahlten Quarzglasmikrophotoreaktors.

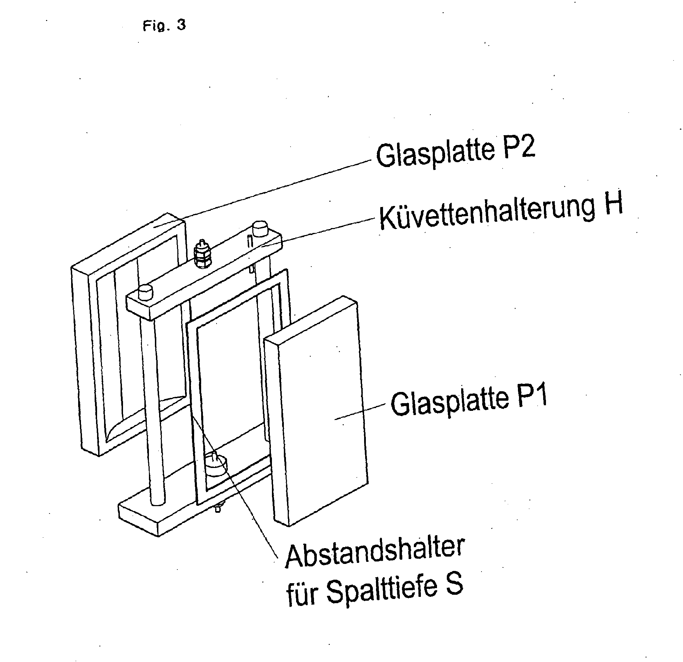

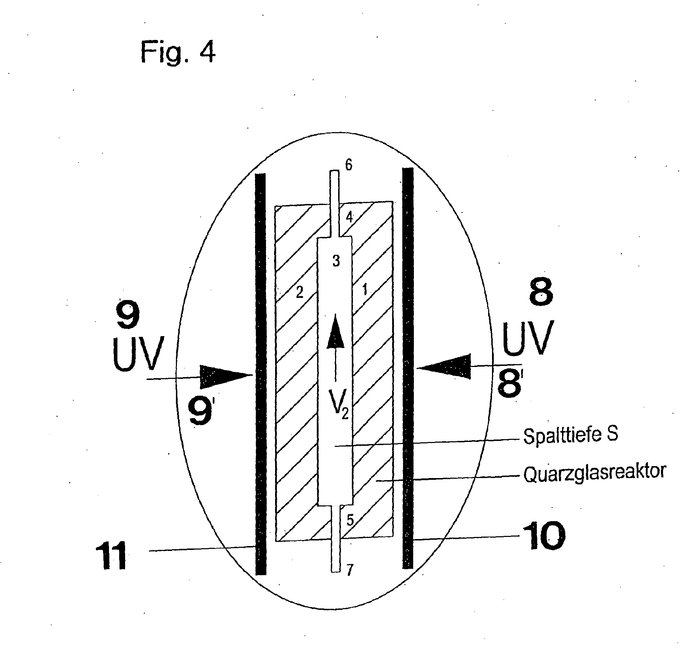

Die schematische Darstellung der Fig. 4 zeigt im Querschnitt die Reaktoreinheit R bestehend aus zwei Quarzglasscheiben (P1 und P2 Fig. 3) **1, 2,** stoffbündig verbunden mit einem Abstandshalter **4, 5** für die Spalttiefe S, sowie zwei Spektralfilter F. Das eintretende UV-Licht **8, 9** wird von den beiden Pfeilen dargestellt. Im Reaktor läuft ein Fluidstrom mit einer Durchflussgeschwindigkeit v2 in Richtung des schwarzen Pfeils im durch **3** bezeichneten Hohlraum. Es versteht sich, dass der Fluidstrom eine Eintrittszone durchlaufen kann, bevor er in die bestrahlte Zone kommt.

## Patentansprüche

1. Verfahren zur Herstellung von 10-Hydroxycamptothecin oder 7-Alkyl-10-hydroxycamptothecin, mit den Schritten
**A** Bereitstellung eines kontinuierlichen Fluidstroms mit Dicke **d** einer Lösung von Camptothecin-*N*-oxid oder 7-Alkyl-camptothecin-*N*-oxid, wobei das Lösemittel aus Dimethylformamid oder einer Mischung von 1,4-Dioxan und Dimethylformamid besteht,
**B** Vorbeiführen des Stroms an mindestens einer Lichtquelle, die parallel zur Dicke **d** Licht abstrahlt, wobei **d** bei 10 µm bis 1000 µm liegt.

2. Verfahren nach Anspruch 1, wobei der Fluidstrom rohrförmig oder quaderförmig mit Länge **I**, Breite **b** und Dicke d bereitgestellt wird.

3. Verfahren nach einem der vorstehenden Ansprüche, wobei **d** bei 20 µm bis 500 µm liegt.

4. Verfahren nach Anspruch 3, wobei **d** bei 40 µm bis 100 µm liegt.

5. Verfahren nach Anspruch 1, wobei die Lichtquelle Licht im Bereich von 350 bis 400 nm abstrahlt.

6. Verfahren nach Anspruch 1, wobei die Lichtquelle mindestens eine LED-Quelle mit Licht einer Wellenlänge von 375 oder 385 nm ist.

7. Verfahren nach Anspruch 1, wobei Camptothecin-*N*-oxid oder 7-Alkyl-camptothecin-*N-*oxid in einer Konzentration von 0.1 % bis 3 % in der Lösung vorliegen.

8. Verfahren nach einem der vorstehenden Ansprüche, durchgeführt simultan mit mehreren parallel betriebenen Vorrichtungen ("Numbering up") nach einem der Ansprüche 10-13.

9. Verfahren nach einem der vorstehenden Ansprüche, durchgeführt mit mindestens zwei seriell geschalteten Vorrichtungen nach einem der Ansprüche 10-13.

10. Vorrichtung zum Ausführen des Verfahrens nach Anspruch 1, aufweisend zwei im Abstand **d** von 10 µm bis 1000 µm parallel angeordnete, transparente, ebenen Quarzglas-Platten (1, 2),
zwei den Raum (3) zwischen den Platten (1, 2) an zwei einander gegenüberliegenden Seiten begrenzende Wände (4, 5),
zwei nicht durch die Wände begrenzten, einander gegenüberliegenden Öffnungen (6, 7), zwei senkrecht zu den Platten derart angeordnete Lichtquellen (8, 9),
dass deren Strahlung (8', 9') durch die beiden Platten (1, 2) in den Raum (3) dringt, **dadurch gekennzeichnet, dass** die Platten stoffschlüssig miteinander verbunden sind.

11. Vorrichtung nach Anspruch 10, wobei **d** bei 20 µm bis 500 µm liegt.

12. Vorrichtung nach Anspruch 11, wobei **d** bei 40 µm bis 100 µm liegt.

13. Vorrichtung nach Anspruch 10, bei der zwischen den Lichtquellen (8, 9) und den Quarzglas-Platten (1, 2) Filter (10, 11) zum Herausfiltern unerwünschter Wellenlängenbereiche angeordnet sind.

## Claims

1. Method for producing 10-hydroxycamptothecine or 7-alkyl-10-hydroxycamptothecine, comprising the steps:
**A** providing a continuous fluid stream having a thickness **d** of a solution of camptothecine-*N*-oxide or 7-alkyl-camptothecine-*N*-oxide, whereby the solvent consists of dimethylformamide or a mixture of 1,4-dioxane and dimethylformamide;
**B** guiding the stream past at least one light source that emits light parallel to the thickness **d,** whereby **d** is 10 µm to 1,000 µm.

2. Method according to claim 1, whereby the fluid stream is provided to be tube-shaped or cuboid-shaped having length **I**, width **b**, and thickness **d.**

3. Method according to any one of the preceding claims, whereby d is 20 µm to 500 µm.

4. Method according to claim 3, whereby **d** is 40 µm to 100 µm.

5. Method according to claim 1, whereby the light source emits light in the range from 350 to 400 nm.

6. Method according to claim 1, whereby the light source is at least one LED source with light of a wavelength of 375 or 385 nm.

7. Method according to claim 1, whereby camptothecine-*N*-oxide or 7-alkyl-camptothecine-*N*-oxide are present in the solution at a concentration of 0.1 % to 3 %.

8. Method according to any one of the preceding claims, carried out simultaneously with multiple devices, operated in parallel ("numbering up"), according to any one of the claims 10-13.

9. Method according to any one of the preceding claims, carried out with at least two devices, operated in series, according to any one of the claims 10-13.

10. Device for carrying out the method according to claim 1, comprising two transparent, planar quartz glass plates (1, 2) that are arranged parallel to each other at a distance **d** of 10 µm to 1,000 µm;
two walls (4, 5) that limit the space (3) between said plates (1, 2) on two opposite sides;
two opposite openings (6, 7) that are not limited by the walls;
two light sources (8, 9) that are arranged perpendicular to the plates such that their radiation (8', 9') penetrates through the two plates (1, 2) into the space (3), **characterised by** the plates being connected to each other in a firmly bonded manner.

11. Device according to claim 10, whereby **d** is 20 µm to 500 µm.

12. Device according to claim 11, whereby **d** is 40 µm to 100 µm.

13. Device according to claim 10, in which filters (10, 11) for filtering out undesired wavelength ranges are arranged between the light sources (8, 9) and the quartz glass plates (1, 2).

## Revendications

1. Procédé de préparation de 10-hydroxycamptothécine ou de 7-alkyl-10-hydroxycamptothécine, comprenant les étapes de
**A** préparation d'un courant fluidique continu d'épaisseur **d** d'une solution de camptothécine-N-oxyde ou de 7-alkyl-camptothécine-N-oxyde, dans lequel le solvant se compose de diméthylformamide ou d'un mélange de 1,4-dioxane et de diméthylformamide,
**B** passage du courant devant au moins une source lumineuse qui émet de la lumière parallèlement à l'épaisseur **d,** dans lequel **d** se situe aux alentours de 10 µm à 1000 µm.

2. Procédé selon la revendication 1, dans lequel le courant fluidique est préparé en forme tubulaire ou de parallélépipède avec une longueur I, une largeur **b** et une épaisseur **d.**

3. Procédé selon l'une quelconque des revendications précédentes, dans lequel **d** se situe aux alentours de 20 µm à 500 µm.

4. Procédé selon la revendication 3, dans lequel d se situe aux alentours de 40 µm à 100 µm.

5. Procédé selon la revendication 1, dans lequel la source lumineuse émet de la lumière dans la plage de 350 à 400 nm.

6. Procédé selon la revendication 1, dans lequel la source lumineuse est au moins une source LED avec de la lumière d'une longueur d'onde de 375 ou 385 nm.

7. Procédé selon la revendication 1, dans lequel le camptothécine-N-oxyde ou le 7-alkyl-camptothécine-N-oxyde est présent dans la solution à une concentration de 0,1 % à 3%.

8. Procédé selon l'une quelconque des revendications précédentes, réalisé simultanément avec plusieurs dispositifs fonctionnant en parallèle (« Numbering up ») selon l'une quelconque des revendications 10 à 13.

9. Procédé selon l'une quelconque des revendications précédentes, réalisé avec au moins deux dispositifs montés en série selon l'une quelconque des revendications 10 à 13.

10. Dispositif pour réaliser le procédé selon la revendication 1, présentant deux plaques en verre quartzeux planes transparentes (1, 2) disposées parallèlement à un écart **d** de 10 µm à 1000 µm,
deux parois (4, 5) limitant l'espace (3) entre les plaques (1, 2) sur deux côtés opposés,
deux ouvertures opposées (6, 7) non limitées par les parois,
deux sources lumineuses (8, 9) disposées perpendiculairement aux plaques de telle sorte
que leur rayonnement (8', 9') pénètre à travers les deux plaques (1, 2) dans l'espace (3), **caractérisé en ce que** les plaques sont reliées ensemble par liaison de matière.

11. Dispositif selon la revendication 10, dans lequel **d** se situe aux alentours de 20 µm à 500 µm.

12. Dispositif selon la revendication 11, dans lequel **d** se situe aux alentours de 40 µm à 100 µm.

13. Dispositif selon la revendication 10, dans lequel des filtres (10, 11) sont disposés entre les sources lumineuses (8, 9) et les plaques en verre quartzeux (1, 2) pour filtrer des plages de longueur d'onde indésirables.
